# EUROPEAN PATENT SPECIFICATION

(11) **EP 1 962 746 B1**
(45) Date of publication and mention of the grant of the patent: **27.04.2011**
(21) Application number: 06809987.8
(22) Date of filing: 20.09.2006
(51) Int. Cl.: A61F 5/01

(54) **ORTHOPEDIC SUPPORT OR ORTHESIS FOR THE HAND, IN PARTICULAR FOR THE THUMB**
ORTHOPÄDISCHE STÜTZE ODER ORTHESE FÜR DIE HAND, INSBESONDERE FÜR DEN DAUMEN
SUPPORT ORTHOPEDIQUE OU ORTHESE DE LA MAIN, EN PARTICULIER DU POUCE

(30) Priority: 06.12.2005 IT VR20050153
(43) Date of publication of application: 03.09.2008
(73) Proprietor: F.G.P. Srl, 37062 Dossobuono (VR) (IT)
(72) Inventor: FERRIGOLO, Moreno, I-37062 Dossobuono (IT); TURRINI, Alberto, I-37062 Castel d'Azzano (IT)
(74) Representative: Sandri, Sandro
(86) International application number: PCT/IT2006/000672
(87) International publication number: WO 2007/066367

(56) References cited:
- US-A- 1 471 948
- US-A- 5 746 707
- US-A1- 2005 145 255
- US-B1- 6 325 772

## Description

### TECHNICAL FIELD

This invention concerns an orthopedic support, or orthesis, for the hand, which allows restraint of a hand that must be immobilized, for example following injury, spraining, dislocation or the like.

More specifically, this invention refers to an orthopedic support for the hand which, with respect to known solutions, has the advantage of being perfectly flexible and therefore adaptable to various situations and conditions in which it is used, as well as being easy to construct and therefore reproducible with very limited costs.

The support according to the invention substantially consists of a fixed type orthesis for the thumb or, if necessary and after appropriate adaptation, for other fingers.

This invention can be applied in the production sector of orthopedic products and braces mainly used in conservative, post-traumatic, rehabilitation and postoperative therapy.

### BACKGROUND ART

It is known that numerous injuries and sprains can affect the hand or the fingers and that the treatment and rehabilitation methods are also numerous.

As far as the fingers are concerned, it is also known that the most traumatic and therefore the most problematic events from the point of view of reconstruction and rehabilitation are fractures, dislocation of the thumb, arthrosis or tendonitis of the thumb, metacarpophalangeal sprains, the after-effects of fractures and of trapeziometacarpal arthrodesia and of thumb surgery, above all in cases of rheumatic disorders and degenerative processes or of atonicity.

In these and in other no less problematic cases fixed or partially fixed thumb ortheses are used, with, for example, a plastic or polished polypropylene structure that may also be equipped with a wrist closing strap and a possibly an elasticated cotton protective cover to be put on before fitting the orthesis.

In other cases, ortheses made from a rigid structure are used, fitted with a pouch in correspondence with the thumb to accommodate an insert that can be modeled after hot water has been inserted in the pouch, using models that respect particular individual requirements.

The orthesis can consist of a rear splint to block the wrist and a heat-adaptable part, contained in a pouch, to immobilize the thumb.

The most common form of orthesis consists of a rigid enclosure, splinted in correspondence with the thumb, which stabilizes the carpometacarpal and metacarpophalangeal joint.

The types of orthesis described above allow the wrist and the thumb to be immobilized in a functional position, also in cases of sprains, tendonitis and radial nerve paralysis.

A first problem encountered with the use of traditional ortheses concerns the fact that once the position of the thumb with respect to the wrist has been set, it is generally no longer possible to change this as the structure is rigid and non-deformable.

Another problem concerns the difficulty in constructing ortheses for the wrist-thumb, which are on one hand resistant to stress and on the other are malleable, so they can adapt to the various and varying conditions that can be encountered as therapy proceeds.

It has also been found that the majority of traditional ortheses are the standardized type and do not adapt to the various problems of the thumb so that once their use in a certain condition has ended it is not possible to use them again for other conditions, especially if they are heat-adaptable type ortheses.

This involves considerable wastage of materials and means, affecting production costs and consequently the retail price.

Document US-A-1471948 discloses a combined wrist and thumb protector and bracing member comprising a wristband, a circlet for surrounding a sprained or injured thumb, and a resilient splint extending from the wristband to the thumb-encircling member. The wristband and the thumb-encircling member are made of leather, while the splint is made of resilient metal.

### DESCRIPTION OF THE INVENTION

This invention proposes to provide an orthopedic support for the hand, which makes it possible to restrain the thumb with respect to the wrist and which is able to eliminate or at least reduce the problems described above.

In particular, the orthesis according to the invention proposes to resolve the problems caused by the limited or non-existent flexibility of the traditional systems of thumb restraint used so far.

The invention also proposes to provide an orthesis for the thumb-wrist that is easy to produce so as to be economically advantageous as well as extremely efficient from the point of view of safety and stability. At the same time it is very light and extremely limited in size, being minimally invasive as it is fixed to the wrist and the thumb, thus limiting its size and the surface area covered.

This is achieved by means of an orthesis for the thumb with the features described in the main claim.

The dependent claims describe advantageous embodiments of the invention.

The proposed aims are achieved, according to the invention, by an orthesis which consists of a substantially metal structure, shaped so that it can be fitted on the lower part of the wrist, extending first along the initial part of the palm and then towards the thumb.

The metal structure is covered with at least one inner layer of fabric or soft cloth and presenting a series of slots arranged so as to accommodate Velcro or similar types of restraining straps.

The orthesis according to the invention thus foresees that the size of the structure allows it to be easily positioned, first on the wrist and then on the thumb.

Once positioned as described above, the orthesis can be secured by means of the Velcro straps it is fitted with.

### DESCRIPTION OF THE DRAWINGS

Other features and advantages of the invention will become evident on reading the following description of one embodiment of the invention, given as a non-binding example, with the help of the accompanying drawings in which:

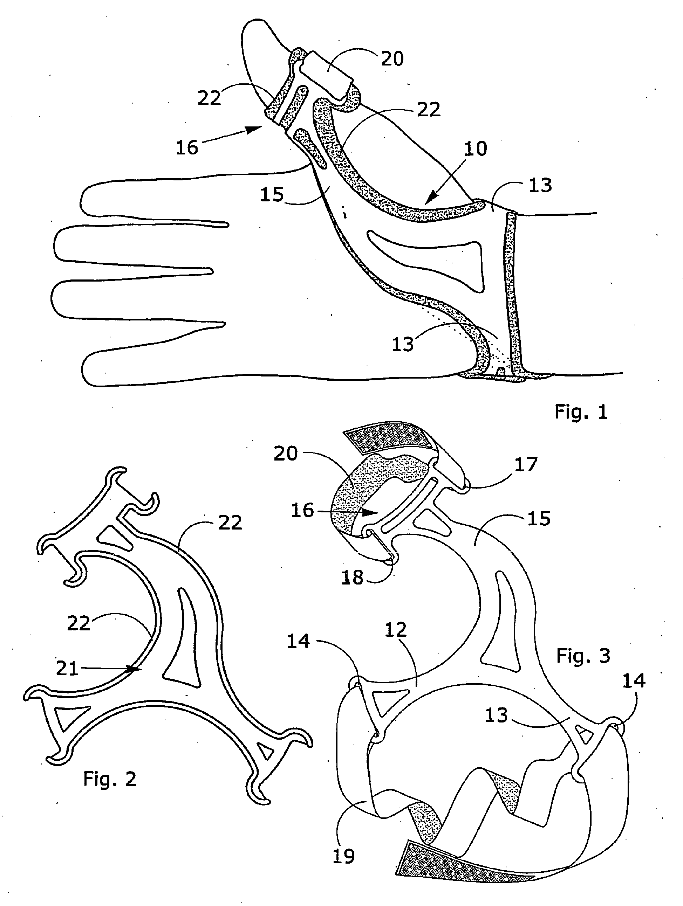

- figure 1 represents a schematic and prospective view of a hand in the open position, on which the orthesis according to the invention has been fitted;
- figure 2 is a schematic plan view of the covered side of the orthesis according to the invention;
- figure 3 is a schematic view of the orthesis according to the invention in the open position.

### DESCRIPTION OF ONE EMBODIMENT OF THE INVENTION

The orthesis according to the invention, mainly but not exclusively used in the orthopedic sector for blocking and anatomically supporting the thumb in a correct position, is indicated overall with the reference number 10 and substantially consists of a rigid support 11, made from metal and being substantially "Y" shaped.

The rigid support 11 has two branches 12 and 13 whose ends present an elongated slot 14 at right angles to the axis of the branches.

The central branch of the structure indicated with 15 presents a substantially "T" shaped head 16 whose ends present slots 17 and 18 .

The central branch 15 of the structure is shaped to follow the anatomical form of the hand, extending along the palm towards the inner side of the thumb.

The orthesis is therefore shaped to follow the hand anatomically and restrain the thumb in the correct position, while the section 15 connected to the wrist section holds the thumb in its anatomical position.

The slots 14 are designed to accommodate a strap 19 which allows the piece to be fixed to the user's wrist, while the slots 17 and 18 allow the insertion of another velcro strap 20 designed to restrain the thumb to the branch 15 of the orthesis.

Each of the straps 19 and 20 is fitted with opening and adjustment means, for example the Velcro type or other type of adjustment fastening.

The described structure is covered internally with a covering 21 made from soft cloth or velvet, shaped to correspond to the metal structure on which it is applied, with the addition of a raised border 22.

The orthesis 11, made from metal or sufficiently rigid and resistant material to adhere to and compress the hand correctly and anatomically starting from the wrist and extending as far as the tip of the thumb, has an elongated and curvilinear shape in accordance with the anatomical shape of the hand.

To use the orthesis according to the invention it is therefore sufficient to apply it to the inner part of the wrist in correspondence with the palm, fitting the two sectors 12 and 13 around the wrist and the central section 15 on the inner part of the thumb.

The strap 19 is then secured around the wrist and the strap 20 around the end of the thumb so that the thumb is completely blocked with respect to the wrist.

The orthesis according to the invention has the advantage of covering only a small surface of the hand and, due to the flexibility and malleability of the metal from which it is made, of being adaptable to the position that the thumb should have with respect to the rest of the hand and the wrist.

According to other embodiments, the orthesis according to the invention can also be constructed for the restraint or blocking of other fingers, by constructing frame models that, instead of extending laterally towards the thumb, extend in a straight line from the wrist to restrain other fingers, such as the index finger, the ring finger, the middle finger or the little finger.

Finally, the orthesis can be constructed with multiple supports to restrain more than one finger at the same time.

The orthesis according to the invention can also naturally be constructed in various sizes for different sized hands.

The invention is described above with reference to a preferred embodiment. It is nevertheless clear that the invention is susceptible to numerous variations that lie within its scope, within the framework of technical equivalents.

## Claims

1. An orthopedic support (10) or orthesis for the hand, which allows restraint of a finger, preferably the thumb on a hand that must be immobilized for example following injury, spraining, dislocation or similar, comprising a substantially "Y" shaped support (11) presenting at least two side branches (12, 13) and one central elongated branch (15), these side and central branches presenting slots (14) for the insertion of straps (19) designed to secure the orthesis to the wrist and at least one finger on the hand, whereby the head (16) of the central elongated branch (15) is substantially "T" shaped and its two ends are fitted with slots (17, 18) for the insertion of another strap (20) for the thumb to be restrained **characterized in that** said support (11) is made from metal or sufficiently rigid, though flexible, material to adhere to and compress the hand correctly and anatomically starting from the wrist and extending as far as the tip of the finger, and it has an elongated and curvilinear shape in accordance with the anatomical shape of the hand, whereby the central branch (15) of the support is shaped and bent to follow the anatomical form of the hand, extending along the palm and the inner side of at least one finger, in particular the thumb.

2. An orthopedic support (10) or orthesis for the hand according to claim 1, **characterized in that** it is shaped to restrain at least one finger, in particular the thumb, in its correct position, while the section (15) connected to the wrist acts as a support in restraining the finger in its anatomical position.

3. An orthopedic support (10) or orthesis for the hand according to any of the foregoing claims, **characterized in that** each of the straps (19, 20) is fitted with opening and adjustment means, for example the Velcro type or other type of adjustment fastening.

4. An orthopedic support (10) or orthesis for the hand according to any of the foregoing claims, **characterized in that** the support is covered internally with a covering (21) made from soft cloth or velvet, shaped to correspond to the metal structure on which it is applied, with the addition of a raised border (22).

5. An orthopedic support (10) or orthesis for the hand according to any of the foregoing claims, **characterized in that** it is constructed with multiple supports in order to restrain more than one finger at the same time.

## Patentansprüche

1. Orthopädische Stütze (10) oder Orthese für die Hand, die die Fixierung eines Fingers ermöglicht, vorzugsweise des Daumens an einer Hand, der zum Beispiel nach einer Verletzung, einer Verstauchung, einer Verrenkung oder etwas ähnlichem ruhig gestellt werden muss, umfassend einen im Wesentlichen "Y"-förmigen Träger (11), der zumindest zwei Seitenarme (12, 13) und einen mittigen länglichen Arm (15) aufweist, wobei diese Seitenarme und der mittige Arm Schlitze (14) aufweisen, in die Riemen (19) eingeführt werden können, die derart gehalten sind, dass die Orthese an dem Handgelenk und an zumindest einem Finger an der Hand festgelegt ist, wobei der Kopf (16) des mittigen länglichen Arms (15) im Wesentlichen "T"-förmig ausgebildet ist und dessen zwei Enden mit Schlitzen (17, 18) versehen sind, um einen weiteren Riemen (20) einzuführen, und zwar für den Daumen, der ruhig gestellt werden soll, **dadurch gekennzeichnet, dass** der Träger (11) aus Metall oder aus einem ausreichend steifen, jedoch flexiblen Material hergestellt ist, um an der Hand anzuhaften und um diese korrekt und anatomisch zu komprimieren, ausgehend von dem Handgelenk und bis zur Spitze des Fingers sich erstreckend, sowie der Träger eine längliche und kurvenförmige Gestalt in Übereinstimmung mit der anatomischen Form der Hand hat, wobei der mittige Arm (15) des Trägers derart geformt und gebogen ist, um der anatomischen Form der Hand zu folgen, sich entlang des Handballens und an der inneren Seite von zumindest einem Finger zu erstrecken, insbesondere des Daumens.

2. Orthopädische Stütze (10) oder Orthese für die Hand nach Anspruch 1, **dadurch gekennzeichnet, dass** sie derart geformt ist, dass zumindest ein Finger in seiner korrekten Position ruhig gestellt ist, insbesondere der Daumen, während der Abschnitt (15), der mit dem Handgelenk verbunden ist, als Träger wirkt, um den Finger in seiner anatomischen Stellung ruhig zu stellen.

3. Orthopädische Stütze (10) oder Orthese für die Hand nach irgendeinem der vorherigen Ansprüche, **dadurch gekennzeichnet, dass** jeder der Riemen (19, 20) mit Öffnungs- und Justiermitteln ausgestattet ist, zum Beispiel mit klettverschlussartigen Typen oder anderen Typen von einstellbaren Befestigungsmitteln.

4. Orthopädische Stütze (10) oder Orthese für die Hand nach irgendeinem der vorherigen Ansprüche, **dadurch gekennzeichnet, dass** der Träger auf der Innenseite mit einer Hüllschicht (21) bedeckt ist, die aus einem weichen Stoff oder Samt hergestellt ist, und die derart geformt ist, dass sie der Metallstruktur entspricht, auf die sie aufgebracht ist, wobei zusätzlich ein verdickter Rand (22) vorgesehen ist.

5. Orthopädische Stütze (10) oder Orthese für die Hand nach irgendeinem der vorherigen Ansprüche, **dadurch gekennzeichnet, dass** sie mit mehreren Trägern ausgebildet ist, um gleichzeitig mehr als nur einen Finger ruhig zu stellen.

## Revendications

1. Support orthopédique (10) ou orthèse pour la main, lequel support orthopédique permet de contraindre un doigt, de préférence le pouce d'une main qui doit être immobilisé par exemple à la suite d'une blessure, d'une foulure, d'une luxation ou similaire, ledit support orthopédique comprenant un support (11) sensiblement en forme de « Y » présentant au moins deux branches latérales (12, 13) et une branche centrale allongée (15), ces branches centrale allongée et latérales présentant des fentes (14) pour l'insertion de sangles (19) conçues pour assurer l'orthèse au poignet et à au moins un doigt de la main, de sorte que la tête (16) de la branche centrale allongée (15) a sensiblement la forme d'un « T » et ses deux extrémités sont dotées de fentes (17, 18) pour l'insertion d'une autre sangle (20) pour le pouce à contraindre, **caractérisé en ce que** ledit support (11) est en métal ou en un matériau suffisamment rigide, tout en étant flexible, pour adhérer à la main, et la comprimer, correctement et anatomiquement en partant du poignet et en s'étendant jusqu'au bout du doigt, et **en ce qu'**il a une forme allongée et curviligne conformément à la forme anatomique de la main, de sorte que la branche centrale (15) du support est conformée et incurvée de manière à épouser la paume et le côté intérieur d'au moins un doigt, notamment le pouce.

2. Support orthopédique (10) ou orthèse pour la main selon la revendication 1, **caractérisé en ce qu'**il est conformé pour contraindre au moins un doigt, notamment le pouce, dans sa position correcte, tandis que la section (15) raccordée au poignet agit comme un support lorsque le doigt est contraint dans sa position anatomique.

3. Support orthopédique (10) ou orthèse pour la main selon l'une quelconque des revendications précédentes, **caractérisé en ce que** chacune des sangles (19, 20) est dotée de moyens d'ouverture et de réglage, par exemple du type Velcro ou un autre type de fixation par réglage.

4. Support orthopédique (10) ou orthèse pour la main selon l'une quelconque des revendications précédentes, **caractérisé en ce que** le support est recouvert du côté intérieur d'un revêtement en tissu ou velours doux, conformé de façon à correspondre à la structure métallique sur laquelle il est appliqué et doté en plus d'un bord relevé (22).

5. Support orthopédique (10) ou orthèse pour la main selon l'une quelconque des revendications précédentes, **caractérisé en ce qu'**il est élaboré avec de multiples supports afin de contraindre plus d'un doigt en même temps.
